(19) Europäisches Patentamt
European Patent Office
Office européen des brevets

(11) Numéro de publication : **0 384 091 B1**

(12) # FASCICULE DE BREVET EUROPEEN

(45) Date de publication du fascicule du brevet : **14.07.93 Bulletin 93/28**

(21) Numéro de dépôt : **89403447.9**

(22) Date de dépôt : **12.12.89**

(51) Int. Cl.[5] : **C07C 69/716,** C07C 67/26, C08F 222/20, C08F 220/28, D04H 1/58

(54) **Nouveaux dérivés du glycérol, leur procédé de préparation, compositions réticulantes en contenant, et leur utilisation dans l'industrie textile.**

(30) Priorité : **23.02.89 FR 8902341**

(43) Date de publication de la demande : **29.08.90 Bulletin 90/35**

(45) Mention de la délivrance du brevet : **14.07.93 Bulletin 93/28**

(84) Etats contractants désignés : **AT BE CH DE ES FR GB GR IT LI LU NL SE**

(56) Documents cités : **Eléments de la technique relevés: néant.**

(73) Titulaire : **SOCIETE FRANCAISE HOECHST TOUR ROUSSEL HOECHST 1 Terrasse Bellini F-92800 Puteaux (FR)**

(72) Inventeur : **Wilhelm, Didier**
**26 Rue Diderot**
**F-92130 Issy les Moulineaux (FR)**
Inventeur : **Cuirassier, Fernand**
**5 Rue de la Légion d'Honneur**
**F-93200 St. Denis (FR)**
Inventeur : **Blanc, Alain**
**21bis Rue Galvanis**
**F-75017 Paris (FR)**

(74) Mandataire : **Rinuy, Santarelli**
**14, avenue de la Grande Armée**
**F-75017 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux dérivés du glycérol, leur procédé de préparation, leur utilisation comme agents de réticulation, les compositions réticulantes ou réticulables en contenant et leur utilisation dans l'industrie textile.

On connaît depuis longtemps des copolymères susceptibles d'être insolubilisés par réticulation. Cette réticulation est généralement obtenue par introduction dans le mélange des monomères à copolymériser d'un monomère comportant un ou plusieurs groupes réactifs tels qu'un groupe carboxyle, vinyle, hydroxyle, oxiranne, carbamoyle éventuellement substitué, carboxyhydroxyméthyle éventuellement éthérifié, comme par exemple le N-méthylolacrylamide, l'acide acrylamidoglycolique, le méthylènebisacrylamide, le diacryloylamino-1,2 dihydroxy-1,2 éthane, le tétraallyloxyéthane, le méthacrylate de glycidyle.

Certains copolymères peuvent être réticulés par simple chauffage, généralement en présence d'un catalyseur acide ou susceptible de libérer un acide, c'est le cas, par exemple, des copolymères acryliques, vinyliques ou acrylovinyliques contenant comme agent de réticulation des amides méthylolés d'acides organiques insaturés tels que les acides acrylique, méthacrylique maléique.

Toutefois, certains de ces monomères réticulants possèdent la propriété de libérer soit au cours de leur polymérisation, soit lors de leur réticulation, des traces de formaldéhyde dont on connait aujourd'hui les inconvénients ; d'autres sont soit insolubles dans l'eau, soit peu réactifs, soit difficilement incorporables dans le copolymère désiré. Or la présente invention a notamment pour objet de fournir de nouveaux agents réticulants obviant à ces inconvénients.

Les nouveaux agents réticulants selon la présente invention sont les dérivés du glycérol de formule générale (I) :

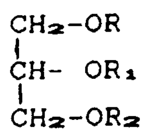

(I)

dans laquelle R représente un groupement allyle, méthylallyle, acryloyle ou méthacryloyle et $R_1$ et $R_2$ soit identiques, représentent un groupement glyoxyloyle, soit différents, représentent un atome d'hydrogène ou un groupement glyoxyloyle.

L'invention a plus particulièrement pour objet les dérivés du glycérol tels que définis ci-dessus, caractérisés en ce que dans ladite formule (I), R représente un groupement allyle, acryloyle ou méthacryloyle et $R_1$ et $R_2$ conservent la signification donnée précédemment.

Parmi ces derniers produits, l'invention a notamment pour objet :

- l'acryloyloxy-3 glyoxyloyloxy-2 propanol-1;
- l'acryloyloxy-1 glyoxyloyloxy-3 propanol-2;
- l'acryloyloxy-1 diglyoxyloyloxy-2,3 propane;
- le méthacryloyloxy-3 glyoxyloyloxy-2 propanol-1 ;
- le méthacryloyloxy-1 glyoxyloyloxy-3 propanol-2 ;
- le méthacryloyloxy-1 diglyoxyloyloxy-2,3 propane;
- l'allyloxy-3 glyoxyloyloxy-2 propanol-1;
- l'allyloxy-1 glyoxyloyloxy-3 propanol-2.

Selon l'invention, les dérivés de formule générale (I) ci-dessus peuvent être préparés par un procédé caractérisé en ce que l'on fait réagir l'acide glyoxylique avec un oxiranne substitué de formule générale (II) :

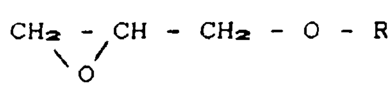

(II)

dans laquelle R a la signification indiquée précédemment, pour obtenir un produit de formule générale (I) tel que défini ci-dessus :

Dans ces conditions préférentielles de mise en oeuvre du procédé de l'invention, le procédé décrit ci-dessus est réalisé de la manière suivante :

La réaction de l'acide glyoxylique avec l'oxiranne substitué de formule générale (II) est effectuée :

. à une température comprise entre 50°C et 120°C, avantageusement aux environs de 80°C ;
. avec un excès d'oxiranne substitué de formule générale (II) ;

. avec de l'acide glyoxylique en solution aqueuse, avantageusement avec une solution aqueuse d'acide glyoxylique à 75 ± 10 % en poids ;

. en présence d'un catalyseur soit dérivé du chrome tel que l'acétylacétonate de chrome, soit de préférence basique tel qu'une amine tertiaire comme la triéthylamine ou la diméthyllaurylamine ;

. en présence éventuellement d'un solvant tel qu'un hydrocarbure aliphatique ou aromatique, comme ...;

. en éliminant en fin de réaction l'excès de l'oxiranne substitué utilisé par lavage du milieu réactionnel dilué à l'eau avec un solvant organique compatible, non miscible à l'eau, de préférence l'oxyde de diéthyle.

Le produit de formule générale (I) peut être identifié grâce à la préparation d'un dérivé caractéristique de sa ou de ses fonctions aldéhydiques tel qu'oxime, O-méthyle oxime, phénylhydrazone, dinitrophénylhydrazone selon les méthodes usuelles de préparation de ces dérivés.

Par exemple, on peut identifier le produit de formule générale (I) cherché par l'isolement de sa ou de ses O-méthyl oximes obtenues en faisant réagir en milieu aqueux, un excès de chlorhydrate de méthoxyamine sur le brut réactionnel dont on a éliminé, au préalable, l'oxiranne de départ non transformé. La ou les O-méthyl oximes ainsi obtenues peuvent être purifiées par des moyens connus en soi tels que distillation, chromatographie, etc.

La présente demande a également pour objet l'utilisation des dérivés de formule générale (I) telle que définie ci-dessus comme agents de réticulation.

A titre d'agents de réticulation, les dérivés de formule (I) selon l'invention peuvent avantageusement entrer dans une composition réticulante et/ou réticulable, renfermant au moins un dérivé du glycérol tel que défini ci-dessus copolymérisable avec au moins un monomère copolymérisable avec ce dérivé.

C'est ainsi que la présence dans ce dérivé du glycérol d'une liaison éthylénique permet sa polymérisation ou sa copolymérisation avec d'autres monomères tout en conservant son activité réticulante. Il est vraisemblable que le ou les groupement(s) glyoxyloyle présent(s) dans ce dérivé du glycérol réagissent sur les groupements hydroxyle des polymères ou des copolymères formés pour constituer des ponts acétals et réticulent ainsi les macromolécules, car ainsi qu'il sera montré plus loin, la composition selon l'invention se comporte différemment d'un simple mélange.

Avantageusement, les monomères que l'on peut copolymériser avec un dérivé de formule (I) selon l'invention pour donner des copolymères pouvant eux-mêmes entrer dans une réaction de réticulation comprennent les monomères acryliques ou méthacryliques tels que les acrylates et méthacrylates d'alkyle en $C_1$-$C_{18}$, les éthers et esters vinyliques tels que l'acétate de vinyle, le propionate de vinyle, le chlorure de vinyle, l'acide (méth)acrylique, et ses sels de métal alcalin ou d'ammonium, l'acrylonitrile, le styrène ou des oléfines substituées telles que l'acide vinylsulfonique, de préférence salifié sous forme de sel de sodium. On peut également utiliser des mélanges de ces monomères.

Cette copolymérisation du dérivé du glycérol de formule générale (I) avec d'autres monomères insaturés peut être réalisée en dispersion, en émulsion, en solution soit par un procédé batch, soit par des procédés semi-continus ou continus, selon des moyens connus, en présence d'un ou plusieurs amorceurs de polymérisation tels qu'un système Redox, un azoïque, un peroxyde et/ou un hydroperoxyde. La proportion de l'agent de réticulation introduite dans le copolymère peut varier dans de grandes proportions de 0,1 à 15 % en poids, de préférence de 0,5 à 5 % en poids par rapport au poids total des monomères mis en oeuvre.

Les copolymères réticulants et/ou réticulables sont obtenus en copolymérisant le dérivé du glycérol utilisé comme agent réticulant préalablement préparé. Le choix des monomères entrant dans la copolymérisation permet de modifier certaines propriétés physiques des substances obtenues après réticulation, comme la souplesse, la dureté, la coloration. L'introduction dans le copolymère de groupes réactifs comme des groupes hydroxyle, carboxyle, carbamoyle ou autres, peut utilement renforcer les propriétés de réticulation. L'effet de réticulation peut également être renforcé par addition de catalyseurs connus comme des acides, des sels ammoniacaux ou des sels d'acides minéraux ou organiques de métaux ayant plusieurs valences, comme par exemple, les chlorures, les nitrates de magnésium, zinc, calcium, aluminium, zirconium.

Pour montrer l'aptitude à la réticulation des compositions réticulantes et/ou réticulables selon l'invention, on a mis en évidence la propriété de ces compositions de donner par simple chauffage des films résistant aux solvants organiques. Le chauffage produit une réticulation au sein même des macromolécules du copolymère ou entre les macromolécules du copolymère.

Les propriétés des compositions réticulantes et/ou réticulables de l'invention les rendent plus particulièrement intéressantes dans l'industrie textile ou dans l'industrie papetière. Ces compositions sont en effet utilisables pour la réalisation de mâts de fibres ou des non-tissés ou pour améliorer les effets de traitements pour rendre infroissables les tissus. Elles permettent notamment d'obtenir des tissus, tissés ou non-tissés, résistant bien aux solvants organiques, particulièrement aux solvants organiques utilisés dans les opérations de nettoyage à sec et d'augmenter la résistance à l'abrasion et l'infroissabilité ainsi que la permanence au lavage des traitements destinés à rendre les tissus infroissables ; elles permettent donc l'obtention de tissus infroissables grâce au traitement d'un tissu contenant des fils ou des fibres cellulosiques au moyen d'une telle composition.

Les exemples suivants sont donnés à titre illustratif et nullement limitatif de l'invention. Dans ces exemples, les contrôles physico-chimiques sont réalisés comme suit :

- l'extrait sec, exprimé en pourcentage pondéral, est déterminé par séchage de 1 g de dispersion pendant 3 heures à 105°C ;
- la viscosité Brookfield est déterminée à 20-22°C avec un viscosimètre Brookfield RVT équipé de l'axe 1 à la vitesse de 50 tr/min ;
- le taux de gonflement, TG, est déterminé à la température ambiante par immersion dans le trichloréthylène d'une éprouvette plane circulaire de 50 mm de diamètre découpée sur un film sec, d'environ 0,5 mm d'épaisseur, de la dispersion à tester. Le TG est mesuré simultanément sur un film non traité thermiquement et sur un film traité pendant 10 minutes à 150°C. Le TG est calculé par la relation TG = $\frac{100\,(df - di)}{df}$ dans laquelle di est le diamètre initial de l'éprouvette et df, le diamètre final ;
- les spectres RMN sont déterminés sur un appareil Brucker AC 200 (50 MHz $^{13}C$ et 200 MHz $^{1}H$). Les déplacements chimiques sont exprimés en ppm par rapport au tétraméthylsilane.

Exemple 1

**Préparation du méthacryloyloxy-1 glyoxylolyoxy-3 propanol-2 et du méthacryloyloxy-3 glyoxyloyloxy-2 propanol-1**

On chauffe sous agitation durant 3 heures à 80°C:

- 300 g (2,11 moles) de méthacrylate de glycidyle;
- 48,8 g (0,53 mole) d'acide glyoxylique monohydraté;
- 5,36 g (53 mmoles) de triéthylamine ;
- 0,15 g de paraméthoxyphénol,

puis le milieu réactionnel est refroidi à la température ambiante. On obtient ainsi une solution huileuse désignée Ac qui est utilisée telle quelle. Afin de caractériser les produits présents dans cette solution Ac, elle est versée dans un mélange de 355 g d'eau et de 355 g d'oxyde de diéthyle. Après décantation, la phase aqueuse est lavée deux fois avec 355 g d'oxyde de diéthyle et la phase éthérée est lavée deux fois avec 355 g d'eau. Des phases éthérées réunies, on isole, après élimination de l'oxyde de diéthyle, 156 g (1,1 mole) de méthacrylate de glycidyle non transformé. Les phases aqueuses réunies sont concentrées sous vide jusqu à un poids total de 1140 g, désignées ci-après solution A. Dans 100 g de cette solution A, on introduit 19 g (0,23 mole) de chlorhydrate de méthoxyamine puis la solution obtenue est abandonnée 2 heures à la température ambiante.

On obtient ainsi une suspension laiteuse qu'on lave deux fois avec 136 g de dichlorométhane, puis les phases chlorométhyléniques sont réunies, séchées sur sulfate de magnésium anhydre et enfin concentrées sous vide. On isole ainsi 7,5 g d'huile jaune que l'on purifie par chromatographie préparative sur une colonne (500X25) remplie de silice de granulométrie 10 micromètres, avec élution avec un mélange hexane-acétate d'éthyle 8/2 en volume, ce qui permet d'isoler d'une part, 5 g (20,4 mmoles) de méthacryloyloxy-1 méthoxyiminoacétoxy-3 propanol-2 et, d'autre part, 2 g (8,15 mmoles) de méthacryloyloxy-3 méthoxyiminoacétoxy-2 propanol-1 caractérisés par leurs spectres $RMN_{1}{}^{H}$ enregistré dans le chloroforme deutéré.

**Méthacryloyloxy-1 méthoxyiminoacétoxy-3 propanol-2** (mélange de diastéréoisomères 50/50).

7,49 ppm, s, 1H, CH = N-
6,13 ppm, m, 1H, $CH_2$ = C
5,60 ppm, m, 1H, $CH_2$ = C
4,39-4,16 ppm, m, 5H $-CH_2$ - CH - $CH_2$-
4,04 ppm, s, 3H, $OCH_3$
2,7 ppm, s, 1H, OH
1,93 ppm, m, 3H, $CH_3$ - C=

**Méthacryloyloxy-3 méthoxyiminoacétoxy-2 propanol-1** (mélanges d'isomères)

| | isomères | |
|---|---|---|
| | majoritaire | minoritaire |
| -CH = N, s, 1H | 7,46 ppm | 7,49 ppm |
| $CH_2$ = C, m, 1H | 6,11 ppm | 6,09 ppm |
| $CH_2$ = C, m, 1H | 5,58 ppm | 5,58 ppm |
| -CH-O-CO-, quintuplet, 1H, J = 4,9 Hz | 5,23 ppm | 5,30 ppm |
| -$CH_2$-O-CO-, m, 2H | 4,48 ppm | 4,42-4,3 ppm |
| -$OCH_3$, s, 3H | 4,02 ppm | 4,02 ppm |
| -$CH_2OH$, d, 2H, J = 4,9 Hz | 3,78 ppm | 3,80 ppm |
| -OH, s, 1H | 2,60 ppm | 2,50 ppm |
| $CH_3$-C=, m, 3H | 1,91 ppm | 1,91 ppm |

En conséquence, la solution A contient au moins 50,28 g (0,23 mole) de méthacryloyloxy-1 glyoxyloyloxy-3 propanol-2 et 20,1 g (93 mmoles) de méthacryloyloxy-3 glyoxyloyloxy-2 propanol-1 soit un rendement de 61 % de la théorie calculée par rapport à l'acide glyoxylique mis en oeuvre.

Exemple 2

En opérant comme dans l'exemple 1 au départ de 270,2 g (2,11 moles) d'acrylate de glycidyle, 48,8 g (0,53 mole) d'acide glyoxylique monohydraté, 5,36 g (53 mmoles) de triéthylamine et de 0,15 g de paraméthoxyphénol, on obtient un mélange d'acryloyloxy-1 glyoxyloyloxy-3 propanol-2 et d'acryloyloxy-3 glyoxyloyloxy-2 propanol-1 avec un rendement supérieur à 65 % de la théorie calculée par rapport à l'acide glyoxylique mis en oeuvre.

Ces produits ont été caractérisés par isolement de leur O-méthyl oxime en utilisant le procédé décrit dans l'exemple 1.

**Acryloyloxy-1 méthoxyiminoacétoxy-3 propanol-2**

| Microanalyse | | C % | H % | N % |
|---|---|---|---|---|
| $C_9H_{13}NO_6$ | calculés | 46,75 | 5,7 | 6,05 |
| PM = 231,2 | trouvés | 46,5 | 5,8 | 6,2 |

Analyse physique : RMN[1]H ($CDCl_3$)
7,51 ppm, s, 1H, CH = N
6,44 ppm, dd, 1H, J=1,8 Hz et J=17 Hz, $CH_2$ =C
6,13 ppm, dd, 1H, J=17 Hz et J=10,3 Hz, CH - CO
5,87 ppm, dd, 1H, J=1,8 Hz et J=10,3 Hz, $CH_2$=C
4,29-4,20 ppm, m, 5H,

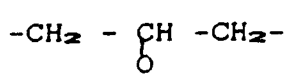

4,05 ppm, s, 3H, -$OCH_3$
2,7 ppm, s, 1H, -OH

**Acryloyloxy-3 méthoxyiminoacétoxy-2 propanol-1**

Analyse physique -RMN[1]H ($CDCl_3$)

| | isomères | |
|---|---|---|
| | majoritaire | minoritaire |
| -CH=N, s, 1H | 7,49 | 7,51 |
| $CH_2$=C, dd, 1H, J=1,8 Hz et J=17 Hz | 6,44 | 6,43 |
| =CH-CO, dd, 1H, J=17 Hz et J=10,3 Hz | 5,87 | 5,865 |
| CH-OCO, quintuplet, 1H, J=4,9 Hz | 5,23 | 5,30 |
| $COOCH_2$, m, 2H | 4,53-4,43 | 4,46-4,40 |
| $OCH_3$, s, 3H | 4,06 | 4,06 |
| OH, d, 2H, J=4,9 Hz | 3,83 | 3,83 |

Exemple 3

En opérant comme dans l'exemple 1 au départ de 300 g (2,63 moles) d'oxyde d'allyle et de glycidyle, de 60,5 g (0,6575 mole) d'acide glyoxylique monohydraté, de 6,6 g (66 mmoles) de triéthylamine et de 150 mg de paraméthoxyphénol, on obtient un mélange d'allyloxy-1 glyoxyloyloxy-3 propanol-2 et d'allyloxy-3 glyoxyloyloxy-2 propanol-1 avec un rendement supérieur à 70 % de la théorie calculée par rapport à l'acide glyoxylique mis en oeuvre.

Ces produits ont été identifiés par isolement de leur O-méthyl oxime selon le procédé décrit dans l'exemple 1. Les caractéristiques physiques de ces oximes sont données ci-après.

**Allyloxy-1 méthoxyiminoacétoxy-3 propanol-2**

Analyse physique RMN[1]H ($CDCl_3$)
7,47 ppm, s, 1H, CH=N
5,90 ppm, m, 1H, =CH-C
5,22 ppm, m, 2H, $CH_2$ =
4,29 ppm, partie AB d'un système ABX, 2H, $J_{AB}$ = 11,5 Hz
$J_{AX}$ = 4,9 Hz, $J_{BX}$ = 6 Hz, O-$CH_2$
4,05 ppm, m, 1H, CH-O
4,02 ppm, s, 3H, $OCH_3$
3,98 ppm, m, 2H, $CH_2$ allylique
3,48 ppm, partie AB d'un système ABX, 2H, $J_{AB}$ = 9,8 Hz
$J_{AX}$ = 4,5 Hz, $J_{BX}$ = 5,8 Hz, O-$CH_2$
2,8 ppm, s, 1H, OH.

**Allyloxy-3 méthoxyiminoacétoxy-2 propanol-1**

Analyse physique RMN[1]H ($CDCl_3$)
7,46 ppm, s, 1H, -CH=N
5,78 ppm, m, 1H, =CH-
5,17 ppm, m, 3H, $CH_2$= et CHOCO
4,0 ppm, s, 3H, $OCH_3$
3,95 ppm, m, 2H, =C-$CH_2$ - O
3,79 ppm, d, 2H, -$CH_2$-OH

3,62 ppm, d, 2H, O- $CH_2$ -CH-
2,95 ppm, s, 1H, OH

Exemple 4

On prépare une solution, désignée $S_{14}$, en dissolvant sous agitation, à la température ambiante, dans 199,6 g d'eau distillée :

- 16,1 g d'une solution aqueuse à 28 % en poids du sel de sodium du lauryléthersulfate éthoxylé avec 3 à 4 moles d'oxyde d'éthylène ;
- 33,8 g d'une solution aqueuse à 20 % en poids d'alcool oléocétylique à 25 moles d'oxyde d'éthylène ;
- 1 g d'hydrogénocarbonate de sodium ;
- 1,2 g de peroxodisulfate de potassium ;
- 2,61 g de méthacrylamide.

Simultanément, on prépare une solution, désignée $S_{24}$, en mélangeant sous agitation, à la température ambiante :

- 192,7 g d'acrylate de n-butyle ;
- 245,85 g de méthacrylate de méthyle ;
- 2,25 g d'acide acrylique ;
- 21,10 g de la solution Ac préparée à l'exemple 1.

Dans un réacteur de polymérisation, on chauffe à 83°C, 250 g d'eau distillée, puis sous agitation, à cette température, on introduit en 4 heures, la solution obtenue en mélangeant extemporanément les deux solutions précédentes, puis l'introduction terminée, on maintient le milieu réactionnel sous agitation pendant une heure à 83 ± 1°C et ensuite on le refroidit à la température ambiante.

Après filtration sur un tamis d'ouverture de mailles de 0,16 mm, on obtient une dispersion aqueuse, fluide, d'aspect laiteux, présentant un pH de 3,95, un extrait sec de 47,76 %, une viscosité Brookfield de 50 mPa.s, une taille de particules (moyenne d'ordre z) de 172 nm, un taux de gonflement, TG, de 122 % et un taux de gonflement de 110 % après traitement thermique.

Exemple 5

On prépare une solution, désignée $S_{15}$, en dissolvant sous agitation, à la température ambiante, dans 273,6 g d'eau distillée :

- 3,6 g d'une solution aqueuse à 50 % en poids de sel de sodium d'éther sulfate de tributylphénol éthoxylé avec 7 moles d'oxyde d'éthylène ;
- 4,5 g d'une solution aqueuse à 20 % en poids de nonylphénol éthoxylé avec 30 moles d'oxyde d'éthylène ;
- 0,9 g de nonylphénol éthoxylé avec 6 moles d'oxyde d'éthylène ;
- 1 g d'hydrogénocarbonate de sodium ;
- 2,61 g de méthacrylamide ;
- 1,35 g de peroxodisulfate de potassium.

Simultanément, on prépare une solution, désignée $S_{25}$, en mélangeant sous agitation, à la température ambiante :

- 232,9 g de styrène ;
- 205,7 g d'acrylate de n-butyle ;
- 2,25 g d'acide acrylique ;
- 21,10 g de la solution Ac préparée à l'exemple 1.

Dans un réacteur de polymérisation, on chauffe à 82°C, sous agitation :

- 250 g d'eau distillée ;
- 0,45 g d'une solution aqueuse à 50 % en poids de sel de sodium d'éthersulfate de tributylphénol éthoxylé avec 7 moles d'oxyde d'éthylène,

puis on introduit en 4 heures, sous agitation, en maintenant la température à 84 ± 1°C, la solution obtenue en mélangeant extemporanément les solutions $S_{15}$ et $S_{25}$. L'introduction terminée, on maintient le milieu réactionnel une heure à 80°C sous agitation puis on le refroidit à la température ambiante et enfin, on le filtre sur un tamis d'ouverture de mailles de 0,16 mm. On obtient ainsi une dispersion fluide, d'aspect laiteux, présentant un pH de 3,35, un extrait sec de 44,5 %, une viscosité Brookfield de 38 mPa.s, une taille des particules (moyenne d'ordre z) de 230 nm, un taux de gonflement de 114 % et un taux de gonflement de 110 % après traitement thermique.

Exemple 6

On prépare une solution, désignée $S_{16}$, en dissolvant sous agitation à la température ambiante dans 209,7 g d'eau distillée :

- 27 g d'une solution aqueuse à 25 % en poids d'alcanesulfonate de sodium linéaire en $C_{14}$-$C_{18}$ ;
- 11,25 g d'une solution aqueuse à 20 % en poids de nonylphénol éthoxylé avec 30 moles d'oxyde d'éthylène;
- 11,25 g d'une solution aqueuse à 20 % en poids d'alcool oléocétylique éthoxylé avec 25 moles d'oxyde d'éthylène ;
- 0,68 g d'acétate de sodium ;
- 0,90 g de peroxodisulfate de potassium ;
- 4,5 g d'une solution aqueuse à 29,7 % en poids de vinylsulfonate de sodium ;
- 2,61 g de méthacrylamide.

Simultanément, on prépare une solution, désignée $S_{26}$, en mélangeant sous agitation, à la température ambiante :

- 137,9 g d'acétate de vinyle ;
- 301,5 g d'acrylate de n-butyle ;
- 21,1 g de la solution Ac préparée à l'exemple 1.

Dans un réacteur de polymérisation, on chauffe à 80°C, 250 g d'eau distillée, puis a cette température sous agitation, on introduit successivement, en une seule fois, une solution aqueuse contenant 0,45 g de peroxodisulfate de potassium dans 10 g d'eau, puis une minute après, une solution aqueuse contenant 0,5 g de métabisulfite de sodium dans 10 g d'eau et enfin, on introduit en 4 heures sous agitation, en maintenant la température à 84°C, la solution obtenue en mélangeant extemporanément les solutions précédentes $S_{16}$ et $S_{26}$. En fin d'introduction, on maintient le milieu réactionnel une heure sous agitation à 84°C puis on le refroidit à la température ambiante et enfin on le filtre sur un tamis d'ouverture de mailles de 0,16 mm. On obtient ainsi une dispersion fluide, d'aspect laiteux, présentant un pH de 3,2, une viscosité Brookfield de 34 mPa.s, une taille de particules (moyenne d'ordre z) de 264 nm, un taux de gonflement de 130 % et un taux de gonflement après traitement thermique de 110 %.

Exemple 7 de comparaison

On prépare une solution désignée $S_{17}$, en dissolvant sous agitation à la température ambiante dans 209,7 g d'eau distillée :

- 27 g d'une solution aqueuse à 25 % en poids d'alcanesulfonate de sodium linéaire en $C_{14}$-$C_{18}$ ;
- 11,25 g d'une solution aqueuse à 20 % en poids de nonylphénol éthoxylé avec 30 moles d'oxyde d'éthylène;
- 11,25 g d'une solution aqueuse à 20 % en poids d'alcool oléocétylique éthoxylé avec 25 moles d'oxyde d'éthylène ;
- 0,68 g d'acétate de sodium ;
- 0,90 g de peroxodisulfate de potassium ;
- 4,5 g d'une solution aqueuse à 29,7 % en poids de vinylsulfonate de sodium ;
- 2,61 g de méthacrylamide.

Simultanément, on prépare une solution, désignée $S_{27}$, en mélangeant sous agitation, à la température ambiante :

- 137,9 g d'acétate de vinyle ;
- 301,5 g d'acrylate de n-butyle ;
- 4,4 g (31 mmoles) de méthacrylate de glycidyle.

Dans un réacteur de polymérisation, on chauffe a 80°C, 250 g d'eau distillée, puis à cette température sous agitation, on introduit successivement, en une seule fois, une solution aqueuse contenant 0,45 g de peroxodisulfate de potassium dans 10 g d'eau, puis une minute après, une solution aqueuse contenant 0,5 g de métabisulfite de sodium dans 10 g d'eau et enfin, on introduit en 4 heures, sous agitation, en maintenant la température à 84°C, la solution aqueuse obtenue en mélangeant extemporanément les solutions précédentes $S_{17}$ et $S_{27}$. En fin d'introduction, on maintient le milieu réactionnel une heure sous agitation à 84°C. On introduit ensuite sous agitation :

- 2,295 g (31 mmoles) d'acide glyoxylique monohydraté,

puis on maintient le milieu réactionnel sous agitation pendant une heure à 83 ± 1°C avant de le refroidir à la température ambiante et enfin on le refroidit à la température ambiante et on le filtre sur un tamis d'ouverture de mailles de 0,16 mm. On obtient ainsi une dispersion fluide d'aspect laiteux, donnant un film soluble

dans le trichloréthylène, ce qui montre l'absence de réticulation. On n'observe pas, non plus, de thermoréticulation lorsqu'on chauffe ce film à 150°C.

Exemple 8 de comparaison

On reproduit l'exemple 7 de comparaison en remplaçant le méthacrylate de glycidyle par une quantité équivalente de méthacrylate de dihydroxy-2,3 propyle, soit 4,96 g (31 mmoles).

En fin de réaction, on obtient une dispersion fluide, d'aspect laiteux, donnant un film soluble dans le trichloréthylène, ce qui montre l'absence de réticulation. On n'observe pas, non plus, de thermoréticulation-lorsqu 'on chauffe ce film à 150°C.

## Revendications

### Revendications pour les Etats contractants suivants : AT, BE, CH, DE, FR, GB, GR, IT, LI, LU, NL, SE

**1.** Dérivés du glycérol de formule générale (I) :

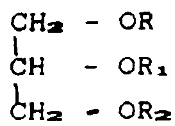

dans laquelle R représente un groupement allyle, méthylallyle, acryloyle ou méthacryloyle, et $R_1$ et $R_2$ soit identiques, représentent un groupement glyoxyloyle, soit différents, représentent un atome d'hydrogène ou un groupement glyoxyloyle.

**2.** Dérivés du glycérol selon la revendication 1, caractérisés en ce que dans la formule (I), R représente un groupement allyle, acryloyle ou méthacryloyle.

**3.** L'acryloyloxy-3 glyoxyloyloxy-2 propanol-1.

**4.** L'acryloyloxy-1 glyoxyloyloxy-3 propanol-2.

**5.** Le méthacryloyloxy-3 glyoxyloyloxy-2 propanol-1.

**6.** Le méthacryloyloxy-1 glyoxyloyloxy-3 propanol-2.

**7.** L'allyloxy-3 glyoxyloyloxy-2 propanol-1.

**8.** L'allyloxy-1 glyoxyloyloxy-3 propanol-2.

**9.** Procédé de préparation des produits de formule générale (I) telle que définie à la revendication 1, caractérisé en ce que l'on fait réagir l'acide glyoxylique avec un oxiranne substitué de formule générale (II) :

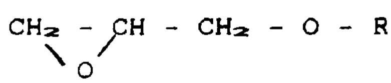

(II)

dans laquelle R a la signification donnée à la revendication 1.

**10.** Procédé selon la revendication 9, caractérisé en ce que l'on fait réagir l'acide glyoxylique avec un oxiranne substitué de formule générale (II) en présence d'un catalyseur basique.

**11.** Utilisation des dérivés de formule générale (I) telle que définie à la revendication 1 comme agents de réticulation.

**12.** Composition réticulante et/ou réticulable, caractérisée en ce qu'elle renferme au moins un dérivé du glycérol selon la revendication 1 copolymérisé avec au moins un monomère copolymérisable avec ce dérivé.

**13.** Composition selon la revendication 12, caractérisée en ce que le monomère copolymérisable est choisi dans le groupe des monomères acryliques ou méthacryliques, des éthers et des esters vinyliques, de l'acide acrylique et de l'acide méthacrylique, de l'acrylonitrile, du styrène, des oléfines et de leurs mélanges.

**14.** Matériau non tissé, caractérisé en ce qu'il est obtenu a partir de fibres naturelles ou synthétiques ou de leur mélange au moyen d'une composition selon l'une quelconque des revendications 12 ou 13.

**15.** Tissu infroissable, caractérisé en ce qu'il est obtenu par traitement d'un tissu contenant des fils ou des fibres cellulosiques au moyen d'une composition selon l'une quelconque des revendications 12 ou 13.

**Revendications pour l'Etat contractant suivant : ES**

**1.** Procédé de préparation d'un dérivé du glycérol de formule générale (I) :

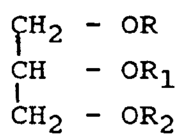

(I)

dans laquelle R représente un groupement allyle, méthylallyle, acryloyle ou méthacryloyle, et $R_1$ et $R_2$ soit identiques, représentent un groupement glyoxyloyle, soit différents, représentent un atome d'hydrogène ou une groupement glyoxyloyle caractérisé en ce que l'on fait réagir l'acide glyoxylique avec un oxiranne substitué de formule générale (II) :

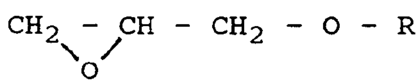

(II)

dans laquelle R a la signification déjà indiquée.

**2.** Procédé selon la revendication 1, caractérisé en ce que l'on fait réagir l'acide glyoxylique avec une oxiranne substitué de formule générale (II) en présence d'un catalyseur basique.

**3.** Procédé selon la revendication 1 ou 2, caractérisé en ce que l'on prépare l'acryloyloxy-3 glyoxyloyloxy-2 propanol-1.

**4.** Procédé selon la revendication 1 ou 2, caractérisé en ce que l'on prépare l'acryloyloxy-1 glyoxyloyloxy-3 propanol-2.

**5.** Procédé selon la revendication 1 ou 2, caractérisé en ce que l'on prépare le méthacryloyloxy-3 glyoxyloyloxy-2 propanol-1.

**6.** Procédé selon la revendication 1 ou 2, caractérisé en ce que l'on prépare le méthacryloyloxy-1 glyoxyloyloxy-3 propanol-2.

**7.** Procédé selon la revendication 1 ou 2, caractérisé en ce que l'on prépare l'allyloxy-3 glyoxyloyloxy-2 propanol-1.

**8.** Procédé selon la revendication 1 ou 2, caractérisé en ce que l'on prépare l'allyloxy-1 glyoxyloyloxy-3 propanol-2.

**9.** Utilisation des dérivés de formule générale (I) tels qu'obtenus selon le procédé de l'une des revendications 1 à 8, comme agents de réticulation.

**10.** Composition réticulante et/ou réticulable, caractérisée en ce qu'elle renferme au moins un dérivé du glycérol tel qu'obtenu selon l'une des revendications 1 à 8 copolymérisé avec au moins un monomère copolymérisable avec ce dérivé.

11. Composition selon la revendication 10, caractérisée en ce que le monomère copolymérisable est choisi dans le groupe des monomères acryliques ou méthacryliques, des éthers et des esters vinyliques, de l'acide acrylique et de l'acide méthacrylique, de l'acrylonitrile, du styrène, des oléfines et de leurs mélanges.

12. Matériau non tissé, caractérisé en ce qu'il est obtenu à partir de fibres naturelles ou synthétiques ou de leur mélange au moyen d'une composition selon l'une quelconque des revendications 10 ou 11.

13. Tissu infroissable, caractérisé en ce qu'il est obtenu par traitement d'un tissu contenant des fils ou des fibres cellulosiques au moyen d'une composition selon l'une quelconque des revendications 10 ou 11.

## Patentansprüche

### Patentansprüche für folgende Vertragsstaaten : AT, BE, CH, DE, FR, GB, GR, IT, LI, LU, NL, SE

1. Glycerinderivate der allgemeinen Formel (I):

$$\begin{array}{ll} CH_2 & - OR \\ | & \\ CH & - OR_1 \\ | & \\ CH_2 & - OR_2 \end{array} \qquad (I)$$

worin R eine Allyl-, Methallyl-, Acryloyl- oder Methacryloylgruppe darstellt, und $R_1$ und $R_2$, falls identisch, eine Glyoxyloylgruppe darstellen, oder, falls verschieden, ein Wasserstoffatom oder eine Glyoxyloylgruppe darstellen.

2. Glycerinderivate nach Anspruch 1, dadurch gekennzeichnet, daß in der Formel (I) R eine Allyl-, Acryloyl- oder Methacryloylgruppe darstellt.

3. 3-Acryloyloxy-2-glyoxyloyloxy-1-propanol.

4. 1-Acryloyloxy-3-glyoxyloyloxy-2-propanol.

5. 3-Methacryloyloxy-2-glyoxyloyloxy-1-propanol.

6. 1-Methacrylyoloxy-3-glyoxyloyloxy-2-propanol.

7. 3-Allyloxy-2-glyoxyloyloxy-1-propanol.

8. 1-Allyloxy-3-glyoxyloyloxy-2-propanol.

9. Verfahren zur Herstellung von Produkten der allgemeinen Formel (I), wie in Anspruch I definiert, dadurch gekennzeichnet, daß man Glyoxylsäure mit einem substituierten Oxiran der allgemeinen Formel (II):

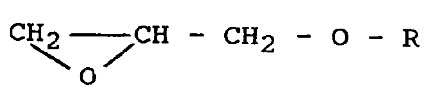

(II)

worin R die in Anspruch 1 angegebene Bedeutung besitzt, reagieren läßt.

10. Verfahren nach Anspruch 9, dadurch gekennzeichnet, daß man Glyoxylsäure mit einem substituierten Oxiran der allgemeinen Formel (II) in Anwesenheit eines basischen Katalysators reagieren läßt.

11. Verwendung der Derivate der allgemeinen Formel (I), wie in Anspruch 1 definiert, als Vernetzungsmittel.

12. Vernetzende und/oder vernetzbare Zusammensetzung, dadurch gekennzeichnet, daß sie wenigstens ein Glycerinderivat nach Anspruch 1, copolymerisiert mit wenigstens einem mit diesem Derivat copolymerisierbaren Monomeren umfaßt.

13. Zusammensetzung nach Anspruch 12, dadurch gekennzeichnet, daß das copolymerisierbare Monomere aus der Gruppe von acrylartigen oder methacrylartigen Monomeren, Vinylethern oder -estern, Acrylsäure und Methacrylsäure, Acrylnitril, Styrol, Olefinen und deren Mischungen ausgewählt ist.

14. Vliesmaterial, dadurch gekennzeichnet, daß es aus nätürlichen oder synthetischen Fasern oder deren Gemischen mit Hilfe einer Zusammensetzung nach einem der Ansprüche 12 oder 13 erhalten worden ist.

15. Knitterfreies Gewebe, dadurch gekennzeichnet, daß es durch Behandlung eines celluloseartige Fäden oder Fasern enthaltenden Gewebes mit einer Zusammensetzung nach einem der Ansprüche 12 oder 13 erhalten worden ist.

**Patentansprüche für folgenden Vertragsstaat : ES**

1. Verfahren zur Herstellung eines Glycerinderivates der allgemeinen Formel (I):

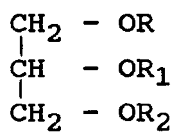
(I)

worin R eine Allyl-, Methallyl-, Acryloyl- oder Methacryloylgruppe darstellt, und $R_1$ und $R_2$, falls identisch, eine Glyoxyloylgruppe darstellen, oder, falls verschieden, ein Wasserstoffatom oder eine Glyoxyloylgruppe darstellen, dadurch gekennzeichnet, daß man Glyoxylsäure mit einem substituierten Oxiran der allgemeinen Formel (II):

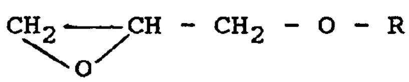
(II)

worin R die zuvor angegebene Bedeutung besitzt, reagieren läßt.

2. Verfahren nach Anspruch 1, dadurch gekennzeichnet, daß man Glyoxylsäure mit einem substituierten Oxiran der allgemeinen Formel (II) in Anwesenheit eines basischen Katalysators reagieren läßt.

3. Verfahren nach Anspruch 1 oder 2, dadurch gekennzeichnet, daß man 3-Acryloyloxy-2-glyoxyloyloxy-1-propanol herstellt.

4. Verfahren nach Anspruch 1 oder 2, dadurch gekennzeichnet, daß man 1-Acryloyloxy-3-glyoxyloyloxy-2-propanol herstellt.

5. Verfahren nach Anspruch 1 oder 2, dadurch gekennzeichnet, daß man 3-Methacryloyloxy-2-glyoxyloyloxy-1-propanol herstellt.

6. Verfahren nach Anspruch 1 oder 2, dadurch gekennzeichnet, daß man 1-Methacrylyoloxy-3-glyoxyloyloxy-2-propanol herstellt.

7. Verfahren nach Anspruch 1 oder 2, dadurch gekennzeichnet, daß man 3-Allyloxy-2-glyoxyloyloxy-1-propanol herstellt.

8. Verfahren nach Anspruch 1 oder 2, dadurch gekennzeichnet, daß man 1-Allyloxy-3-glyoxyloyloxy-2-propanol herstellt.

9. Verwendung von Derivaten der allgemeinen Formel (I), wie sie nach dem Verfahren nach einem der Ansprüche 1 bis 8 erhalten wurden, als Vernetzungsmittel.

10. Vernetzende und/oder vernetzbare Zusammensetzung, dadurch gekennzeichnet, daß sie wenigstens ein

Glycerinderivat, wie nach einem der Ansprüche 1 bis 8 erhalten, copolymerisiert mit wenigstens einem mit diesem Derivat copolymerisierbaren Monomeren umfaßt.

**11.** Zusammensetzung nach Anspruch 10, dadurch gekennzeichnet, daß das copolymerisierbare Monomere aus der Gruppe von acrylartigen oder methacrylartigen Monomeren, Vinylethern oder -estern, Acrylsäure und Methacrylsäure, Acrylnitril, Styrol, Olefinen und deren Mischungen ausgewählt ist.

**12.** Vliesmaterial, dadurch gekennzeichnet, daß es aus natürlichen oder synthetischen Fasern oder deren Gemischen mit Hilfe einer Zusammensetzung nach einem der Ansprüche 10 oder 11 erhalten worden ist.

**13.** Knitterfreies Gewebe, dadurch gekennzeichnet, daß es durch Behandlung eines celluloseartige Fäden oder Fasern enthaltenden Gewebes mit einer Zusammensetzung nach einem der Ansprüche 10 oder 11 erhalten worden ist.

## Claims

**Claims for the following Contracting States : AT, BE, CH, DE, FR, GB, GR, IT, LI, LU, NL, SE**

**1.** Glycerol derivatives of general formula (I):

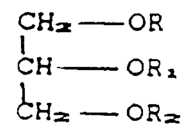

in which R represents an allyl, methylallyl, acryloyl or methacryloyl group, and $R_1$ and $R_2$ are either identical, representing a glyoxyloyl group, or different, representing a hydrogen atom or a glyoxyloyl group.

**2.** Glycerol derivatives according to Claim 1,
**characterised in that** in formula (I) R represents an allyl, acryloyl or methacryloyl group.

**3.** 3-acryloyloxy-2-glyoxyloyloxy-1-propanol.

**4.** 1-acryloyloxy-3-glyoxyloyloxy-2-propanol.

**5.** 3-methacryloyloxy-2-glyoxyloyloxy-1-propanol.

**6.** 1-methacryloyloxy-3-glyoxyloyloxy-2-propanol.

**7.** 3-allyloxy-2-glyoxyloyloxy-1-propanol.

**8.** 1-allyloxy-3-glyoxyloyloxy-2-propanol.

**9.** A preparation process for products of general formula (I) as defined in Claim 1,
**characterised in that** glyoxylic acid is reacted with a substituted oxirane of general formula (II):

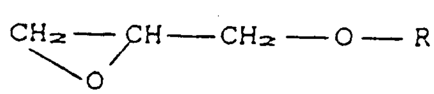 (II)

in which R has the significance given in Claim 1.

**10.** A process according to Claim 9,
**characterised in that** glyoxylic acid is reacted with a substituted oxirane of general formula (II) in the presence of a basic catalyst.

**11.** The use of derivatives of general formula (I) as defined in Claim 1 as cross-linking agents.

12. A cross-linking and/or cross-linkable composition, **characterised in that** it contains at least one glycerol derivative according to Claim 1 copolymerised with at least one monomer copolymerisable with this derivative.

13. A composition according to Claim 12,
**characterised in that** the copolymerisable monomer is chosen from the group of acrylic or methacrylic monomers, vinyl ethers and esters, acrylic acid and methacrylic acid, acrylonitrile, styrene, olefins and mixtures thereof.

14. A non-woven material,
**characterised in that** it is obtained from natural or synthetic fibres or a mixture thereof by means of a composition according to any one of Claims 12 or 13.

15. A crease-resistant material,
**characterised in that** it is obtained by treating a fabric containing cellulose threads or fibres by means of a composition according to any one of Claims 12 or 13.

**Claims for the following Contracting State : ES**

1. A preparation process for a glycerol derivative of general formula (I):

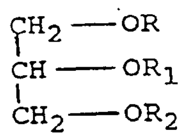

(I)

in which R represents an allyl, methylallyl, acryloyl or methacryloyl group, and $R_1$ and $R_2$ are either identical, representing a glyoxyloyl group, or different, representing a hydrogen atom or a glyoxyloyl group, **characterised in that** glyoxylic acid is reacted with a substituted oxirane of general formula (II):

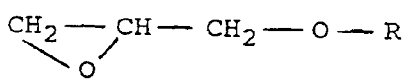

(II)

in which R has the significance already mentioned.

2. A process according to Claim 1,
**characterised in that** glyoxylic acid is reacted with a substituted oxirane of general formula (11) in the presence of a basic catalyst.

3. A process according to Claim 1 or 2,
**characterised in that** 3-acryloyloxy-2-glyoxyloyloxy-1-propanol is prepared.

4. A process according to Claim 1 or 2,
characterised in that 1-acryloyloxy-3-glyoxyloyloxy-2-propanol is prepared.

5. A process according to Claim 1 or 2,
**characterised in that** 3-methacryloyloxy-2-glyoxyloyloxy-1-propanol is prepared.

6. A process according to Claim 1 or 2,
**characterised in that** 1-methacryloyloxy-3-glyoxyloyloxy-2-propanol is prepared.

7. A process according to Claim 1 or 2,
**characterised in that** 3-allyloxy-2-glyoxyloyloxy-1-propanol is prepared.

8. A process according to Claim 1 or 2,
**characterised in that** 1-allyloxy-3-glyoxyloyloxy-2-propanol is prepared.

**9.** The use of derivatives of general formula (I), such as that obtained according to the process of one one Claims 1 to 8, as cross-linking agents.

**10.** A cross-linking and/or cross-linkable composition, **characterised in that** it contains at least one glycerol derivative, such as that obtained according to one of Claims 1 to 8, copolymerised with at least one monomer copolymerisable with this derivative.

**11.** A composition according to Claim 10,
**characterised in that** the copolymerisable monomer is chosen from the group of acrylic or methacrylic monomers, vinyl ethers and esters, acrylic acid and methacrylic acid, acrylonitrile, styrene, olefins and mixtures thereof.

**12.** A non-woven material,
**characterised in that** it is obtained from natural or synthetic fibres or mixtures thereof by means of a composition according to any one of Claims 10 or 11.

**13.** A crease-resistant fabric,
**characterised in that** it is obtained by treating a fabric containing cellulose threads or fibres by means of a composition according to any one of Claims 10 or 11.